# EUROPÄISCHE PATENTSCHRIFT

(11) **EP 0 430 001 B1**
(45) Veröffentlichungstag und Bekanntmachung des Hinweises auf die Patenterteilung: **22.12.1993**
(21) Anmeldenummer: 90121998.0
(22) Anmeldetag: 17.11.1990
(51) Int. Cl.: C07C 205/44, C07C 201/12, C07C 255/56, C07C 253/30

(54) **Verfahren zur Herstellung von Benzaldehyden**
Process for preparing benzaldehydes
Procédé pour la préparation d'aldéhydes benzoiques

(30) Priorität: 01.12.1989 DE 3939759
(43) Veröffentlichungstag der Anmeldung: 05.06.1991
(73) Patentinhaber: BAYER AG, 51368 Leverkusen (DE)
(72) Erfinder: Blank, Heinz Ulrich, Dr., W-5068 Odenthal (DE); Kraus, Helmut, Dr., W-5000 Köln 80 (DE)

(56) Entgegenhaltungen:
- US-A- 3 661 942
- US-A- 3 931 225
- PATENT ABSTRACTS OF JAPAN, unexamined applications, C Field, Band 9, Nr. 138, 13. Juni 1985, THE PATENT OFFICE JAPANESE GOVERNMENT, Seite 123 C 286

## Beschreibung

Die Erfindung betrifft ein Verfahren zur Herstellung von Benzaldehyden, die in mindestens einer der ortho- und para-Stellungen einen elektronenanziehenden Substituenten tragen.

Solche Benzaldehyde besitzen Bedeutung bei der Herstellung von pharmazeutisch wirksamen Präparaten, beispielsweise werden aus o-Nitrobenzaldehyd 1,4-Dihydropyridin-Derivate hergestellt (DE-OS 16 70 827).

Da die meisten klassischen Aldehydsynthesen bei der Synthese von Aldehyden mit elektronenanziehenden Substituenten, beispielsweise bei der Synthese von o-Nitrobenzaldehyd, versagen, existiert bis heute eine umfangreiche Patentliteratur über Spezialverfahren. Einige der beanspruchten Verfahren betreffen in der ersten Stufe die Seitenkettenhalogenierung des zugrundeliegenden o-Nitrotoluols mit anschließender Verseifung (DE-OS 28 42 360) oder Oxidation (DE-OS 27 08 115, DE-OS 29 48 058). Bei der Seitenkettenhalogenierung entstehen jedoch stets auch Kernhalogenierungsprodukte, so daß zur Gewinnung von o-Nitrobenzaldehyd in Pharmaqualität aufwendige Reinigungsschritte notwendig sind.

Die direkte Oxidation von o-Nitrotoluol mit Chrom-(VI)-oxid ist wegen der Abwasserbelastung problematisch. Bei der Verwendung anderer Oxidationsmittel, wie Cer-(IV)-perchlorat (EP 205 173) oder Kobalt(III)-sulfat sprechen die hohe notwendige Verdünnung des Reaktionsansatzes und die geringen erzielbaren Umsätze gegen die Verwirklichung als wirtschaftliches Verfahren.

Auch bei der Oxidation von o-Nitro-styrol mit Sauerstoff in Gegenwart verschiedener Katalysatoren werden nur geringe Umsätze erreicht (DE-OS 28 05 402), während die etwas aufwendigere Verwendung von Ozon bei -20°C bessere Ausbeuten ergibt (DE-OS 28 29 346). Allerdings ist die Herstellung von o-Nitro-styrol umständlich und enthält wiederum einen weniger wünschenswerten Halogenierungsschritt.

Es wurden daher andere Verfahren für den oxidativen Abbau von Verbindungen entwickelt, die durch C-C-Verknüpfung, ausgehend vom kostengünstigen o-Nitrotoluol, hergestellt werden können. Die Oxidation von 2-Nitrophenyl-brenztraubensäure mit Kaliumpermanganat (DE-OS 24 15 061) bzw. mit Hypochlorit und anschließender Verseifung (DE-OS 24 15 062) liefert Ausbeuten von nur 27 bzw. 36 %. Die Umsetzung von Wasserstoffperoxid mit 2-Nitrophenyl-brenztraubensäure-Derivaten (EP-92 267) ergibt 39 bis etwa 50 % o-Nitrobenzaldehyd, jeweils ausgehend vom o-Nitrotoluol.

Es ist auch bereits beschrieben, β-Dimethylamino-2-nitrostyrol mit Natriumhypochlorit bzw. mit 30 %igem Wasserstoffperoxid zu oxidieren (JP 60-25 957 (1985)). Hierzu wurde zunächst von 2-Nitrotoluol ausgegangen, das mit dem ortho-Amid Dimethylformamid-dimethylacetal zum β-Dimethylamino-2-nitrostyrol umgesetzt wurde. Da die beschriebene Oxidation zum Aldehyd in Acetonitril-Wasser-Gemischen durchgeführt wird, muß Dimethylformamid und nicht umgesetztes o-Nitrotoluol abdestilliert werden, was wegen der Zersetzlichkeit der Nitro-styrole nicht ungefährlich ist. Die Ausbeute beträgt bei Verwendung von 30 %igem Wasserstoffperoxid 55 % bzw. 42,5 %, bezogen auf das teure Dimethylformamid-acetal. Bei Verwendung von Hypochlorit und anschließender Verseifung erhält man 68 bzw. 53 % der theoretischen Ausbeute. Eine Nacharbeitung zeigte, daß die Ausbeute bei Verwendung von 70 %igem Wasserstoffperoxid bis zu 80 % der theoretischen Ausbeute gesteigert werden kann; allerdings ist die Verwendung von hochkonzentriertem Wasserstoffperoxid problematisch.

Grundsätzlich ist auch die Oxidation von Enaminen mit Sauerstoff bekannt (Tetrah. Letters 1968, 3271 und 1968, 3267; US 3 661 942), jedoch wurde die Herstellung von Aldehyden nach dieser Methode als unselektiv beschrieben. Die Oxidation von heterocyclischen Enaminen mit O₂ in Gegenwart von CuCl, z.B. von 2-β-Amino-vinyl-5-nitro-imidazol zu 5-Nitro-imidazol-2-carbaldehyd, wird als nicht möglich beschrieben (Ann, 1975, 1465).

Es wurde nun gefunden, daß die weiter unten beschriebenen substituierten β-Amino-styrole direkt mit Sauerstoff zu den anderweitig nur schwierig zugänglichen Benzaldehyden, die in mindestens einer der ortho- und para-Stellungen einen elektronenanziehenden Substituenten tragen, oxidiert werden können, wenn man in Gegenwart einer Cu-Verbindung arbeitet.

Es wurde ein Verfahren zur Herstellung eines Benzaldehyds, der in mindestens einer der ortho- und para-Stellungen einen elektronenanziehenden Substituenten trägt, der Formel 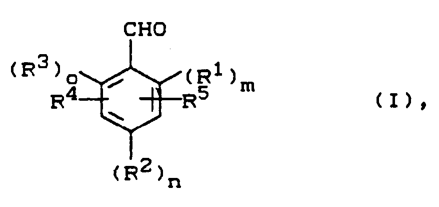
in der
- R¹, R² und R³: unabhängig voneinander Nitro, Cyano, COOR⁶, SO₂-OR⁶ oder SO₂-R⁶ bedeuten,
- R⁴: für Wasserstoff, geradkettiges oder verzweigtes C₁-C₈-Alkyl, geradkettiges oder verzweigtes C₁-C₈-Alkoxy, Phenyl, Benzyl, Cyclopentyl, Cyclohexyl, Fluor, Chlor, Brom oder -N(R⁶,R⁷) steht,
- R⁵: Wasserstoff oder die Aldehydgruppe -CHO bedeutet, wobei
- R⁶ und R⁷: unabhängig voneinander geradkettiges oder verzweigtes C₁-C₄-Alkyl, Benzyl oder Phenyl bedeuten,

wobei weiterhin die genannten cyclischen Substituenten ihrerseits ein- oder zweimal durch Methyl, Ethyl, Methoxy, Ethoxy, Fluor, Chlor oder Brom substituiert sein können und
- m, n und o: unabhängig voneinander den Wert Null oder Eins annehmen können, die Summe m+n+o jedoch auf den Wert Eins oder Zwei beschränkt ist,

gefunden, das dadurch gekennzeichnet ist, daß man ein substituiertes β-Amino-styrol der Formel 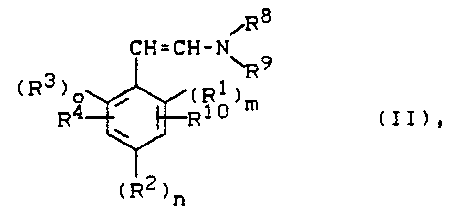
in der
- R¹, R², R³, R⁴, m, n und o: die genannte Bedeutung besitzen,
- R⁸ und R⁹: unabhängig voneinander geradkettiges oder verzweigtes C₁-C₈-Alkyl, C₅-C₇-Cycloalkyl, geradkettiges oder verzweigtes C₂-C₈-Alkenyl, geradkettiges oder verzweigtes C₂-C₈-Alkoxyalkyl, geradkettiges oder verzweigtes C₃-C₈-Alkoxyalkenyl, C₆-C₁₂-Aryl, C₇-C₁₀-Aralkyl oder einen 5-bis 8-gliedrigen gesättigten oder ungesättigten heterocyclischen Ring, dessen Heteroatome ein oder zwei aus der Gruppe von N, O und S sind, bedeuten,

wobei weiterhin
- R⁸ und R⁹: gemeinsam mit dem N-Atom, das sie substituieren, einen 5- bis 8-gliedrigen gesättigten oder ungesättigten, nichtaromatischen N-heterocyclischen Ring bilden können, der ein weiteres Heteroatom aus der Gruppe N, O oder S enthalten kann, und
- R⁸: zusätzlich Wasserstoff bedeuten kann,
- R⁹: zusätzlich die Gruppe 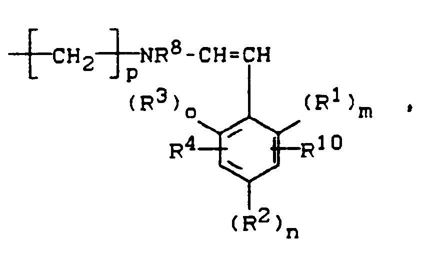 in der p den Wert 2, 3 oder 4 annimmt, bedeuten kann, und
- R¹⁰: Wasserstoff oder die Gruppe 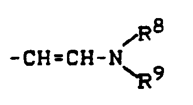 bedeutet,

oder ein Gemisch mehrerer solcher β-Amino-styrole, die sich durch verschiedene Bedeutung von R⁸ und/ oder R⁹ unterscheiden, in der Lösung eines aprotischen, polaren Lösungsmittels bei 0-120°C, bevorzugt bei 20-100°C, besonders bevorzugt bei 30-80°C mit Sauerstoff in Gegenwart einer Cu-Verbindung umsetzt.

Geradkettiges oder verzweigtes C₁-C₈-Alkyl ist beispielsweise Methyl, Ethyl, Propyl, Isopropyl, Butyl, Isobutyl, tert,-Butyl, die isomeren Pentyl-, Hexyl- oder Octylreste. Bevorzugtes Alkyl hat 1-4 C-Atome; Methyl und Ethyl sind besonders bevorzugt.

Geradkettiges oder verzweigtes C₁-C₈-Alkoxy ist beispielsweise Methoxy, Ethoxy, Propoxy, Isopropoxy, Butoxy, Isobutoxy, tert,-Butoxy, die isomeren Pentyloxy-, Hexyloxy- und Octyloxyreste. Bevorzugtes Alkoxy hat 1-4 C-Atome; Methoxy und Ethoxy sind besonders bevorzugt.

C₅-C₇-Cycloalkyl ist beispielsweise Cyclopentyl, Cyclohexyl und Cycloheptyl, bevorzugt Cyclopentyl und Cyclohexyl.

Geradkettiges oder verzweigtes C₂-C₈-Alkenyl ist beispielsweise Vinyl, Propenyl, Allyl, Butenyl, Pentenyl, Hexenyl oder Octenyl sowie deren verzweigte Isomere, in bevorzugter Weise Alkenylreste mit 2-4 C-Atomen.

Geradkettiges oder verzweigtes C₂-C₈-Alkoxyalkyl ist beispielsweise Methoxymethyl, Methoxyethyl, Ethoxymethyl, Ethoxyethyl, Ethoxypropyl, Ethoxybutyl, Ethoxyhexyl, bevorzugt solches mit 2-4 C-Atomen.

Geradkettiges oder verzweigtes C₃-C₈-Alkoxyalkenyl ist beispielsweise Methoxyvinyl, Ethoxyvinyl, Methoxypropenyl, Methoxybutenyl und die höheren, dem Fachmann bekannten Homologen, bevorzugt Methoxyvinyl und Ethoxyvinyl.

C₆-C₁₂-Aryl ist beispielsweise Phenyl, Naphthyl und Biphenylyl, bevorzugt Phenyl.

C₇-C₁₀-Aralkyl ist beispielsweise Benzyl, α- und β-Phenyl-ethyl, Phenyl-propyl und Phenyl-butyl, bevorzugt Benzyl.

5- bis 8-gliedrige gesättigte oder ungesättigte heterocyclische Ringe, die ein oder zwei Heteroatome aus der Gruppe N, O und S enthalten, sind dem Fachmann bekannt und können in ihrer 1-, 2-, 3- oder 4-Position mit dem N-Atom, das sie substituieren, verknüpft sein. Beispiele für solche Ringe sind: Pyrrol, Furan, Thiophen, Pyrrolin, Tetrahydrofuran, Tetrahydrothiophen, Pyrazol, Imidazol, Pyrazolin, Pyrazolidin, Imidazolidin, Oxazol, Thiazol, Oxazolin, Oxazolidin, Thiazolin, Thiazolidin, Pyridin, Pyran, Thiopyran, Piperidin, Dihydropyran, Tetrahydropyran, Dihydrothiopyran, Tetrahydrothiopyran, Pyridazin, Pyrimidin, Pyrazin, Piperazin, Oxazin, Thiazin, Morpholin, Thiomorpholin und andere.

Für den Fall, daß R⁸ und R⁹ gemeinsam mit dem N-Atom, das sie substituierten, einen 5- bis 8-gliedrigen Ring bilden, stellt dieser in jedem Fall einen N-heterocyclischen Ring dar, der gesättigt oder ungesättigt sein kann, jedoch nicht aromatisch ist. Die hierfür aus der obigen Aufzählung in Frage kommenden N-Heterocyclen sind für den Fachmann erkennbar; sie sind stets über ein N-Atom mit der Styrylgruppe verbunden. Bevorzugte Ringe dieser Art sind Pyrrolin, Pyrrolidin, Pyrazolin, Pyrazolidin, Imidazolin, Imidazolidin, Oxazolidin, Thiazolidin, Piperidin, Piperazin, Morpholin und Thiomorpholin; besonders bevorzugt sind hierunter die vollständig gesättigten Ringsysteme.

Unter die umzusetzenden substituierten β-Amino-styrole der Formel (II) fallen auch solche, die eine zweite substituierte β-Amino-vinylgruppe tragen können (R¹⁰) und die demzufolge im Rahmen der erfindungsgemäßen Umsetzung einen Dialdehyd ergeben. In bevorzugter Weise nimmt der Substituent R¹⁰ die Bedeutung Wasserstoff an.

Die umsetzenden substituierten β-Amino-styrole der Formel (II) sind insbesondere gekennzeichnet durch einen oder zwei elektronenanziehende Substituenten, die in ortho-, para-, ortho-para- oder ortho-ortho-Stellung stehen können und deren zugehörige Aldehyde auf anderen Wegen in reiner Form schwer zugänglich sind. Von den obengenannten elektronenanziehenden Substituenten R¹, R² und R³ sind Nitro und Cyano bevorzugt; Nitro ist besonders bevorzugt.

Alle genannten cyclischen Substituenten (Benzolkerne, cycloaliphatische Kerne und heterocyclische Kerne) können ihrerseits ein- oder zweimal durch Methyl, Ethyl, Methoxy, Ethoxy, Fluor, Chlor oder Brom substituiert sein.

Die Herstellung der erfindungsgemäß einzusetzenden substituierten β-Amino-styrole ist bekannt und sei am Beispiel des o-Nitrotoluols als Ausgangsverbindung mit Hilfe des Bis-(dimethylamino)-ethoxrnethan formelmäßig wie folgt beschrieben:

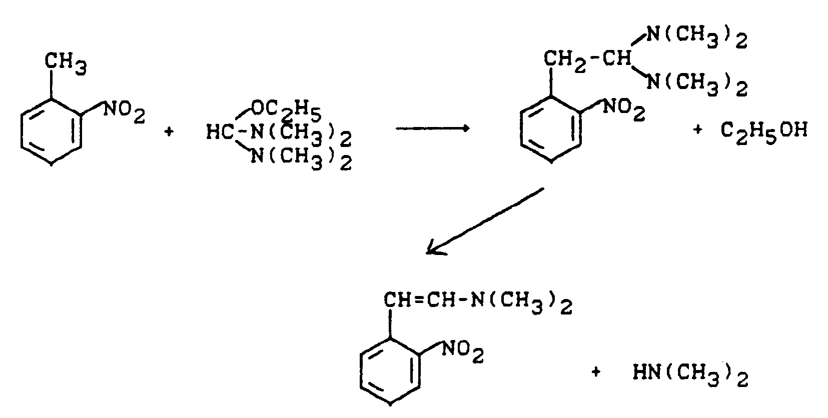

Es ist weiter bekannt, daß die oben formelmäßig dargestellte Herstellung von substituierten β-Amino-styrolen auch in Gegenwart eines sekundären Amins durchgeführt werden kann, wobei ein Aminaustausch stattfinden kann. So kann in dem obigen Formelbeispiel in Gegenwart von Pyrrolidin das folgende Gemisch entstehen, wobei die Spaltprodukte nicht in die Formelgleichung aufgenommen wurden:

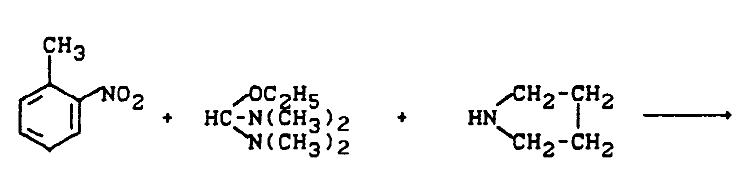

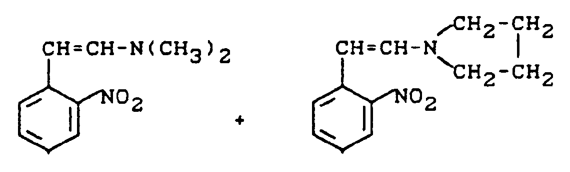

Die erfindungsgemäße, Cu-katalysierte Oxidation der substituierten β-Amino-styrole läßt sich dann beispielhaft wie folgt darstellen:

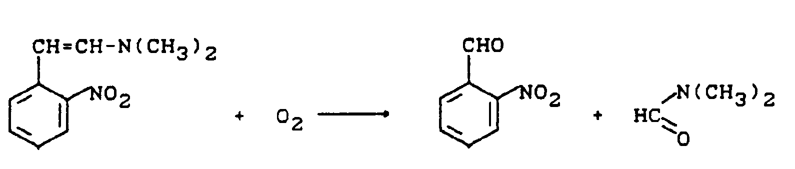

In bevorzugter Weise werden substituierte β-Amino-styrole der Formel 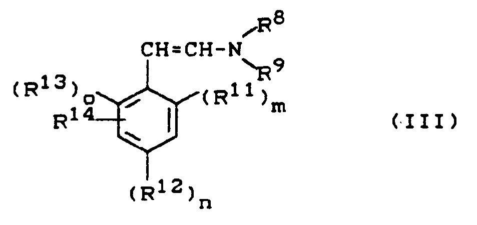
umgesetzt, in der
- R⁸, R⁹, m, n und o: die obengenannte Bedeutung haben,
- R¹¹, R¹² und R¹³: unabhängig voneinander für Nitro oder Cyano stehen und
- R¹⁴: Wasserstoff, geradkettiges oder verzweigtes c₁-C₄-Alkoxy, Fluor, Chlor oder Brom bedeutet.

In besonders bevorzugter Weise werden im erfindungsgemäßen Verfahren substituierte β-Amino-styrole der Formel 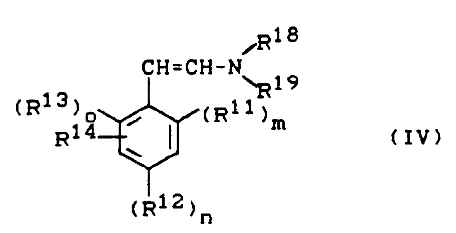
umgesetzt, in der
- R¹¹, R¹², R¹³, R¹⁴, m, n und o: die obengenannte Bedeutung besitzen und
- R¹⁸ und R¹⁹: unabhängig voneinander geradkettiges oder verzweigtes C₁-C₈-Alkyl, Phenyl, Benzyl, Cyclohexyl oder einen 5- bis 6-gliedrigen gesättigten heterocyclischen Ring, der als Heteroatom ein N oder O enthält, bedeuten,

wobei weiterhin
- R¹⁸ und R¹⁹: gemeinsam mit dem N-Atom, das sie substituieren, einen 5- bis 6-gliedrigen N-heterocyclischen Ring bilden können, der ein weiteres Heteroatom aus der Gruppe N und O enthalten kann.

In ganz besonders bevorzugter Weise werden im erfindungsgemäßen Verfahren substituierte β-Amino-styrole der Formel 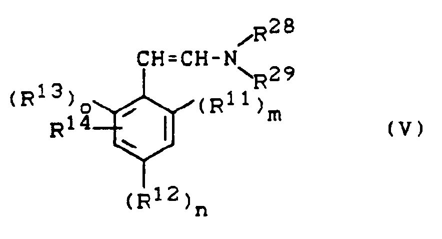
umgesetzt, in der
- R¹¹, R¹², R¹³, R¹⁴, m, n und o: die obengenannte Bedeutung besitzen und
- R²⁸ und R²⁹: unabhängig voneinander geradkettiges oder verzweigtes C₁-C₄-Alkyl, Benzyl oder Cyclohexyl bedeuten,

wobei weiterhin
- R²⁸ und R²⁹: gemeinsam mit den N-Atom das sie substituieren, den Pyrrolidinring, den Oxazolidinring, den Piperidinring, den Piperazinring oder den Morpholinring bilden können.

In weiterhin ganz besonders bevorzugter Weise werden im erfindungsgemäßen Verfahren substituierte β-Amino-2-nitro-styrole der Formel 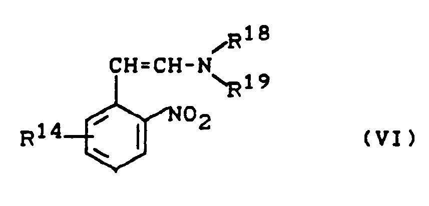
umgesetzt, in der
R¹⁴, R¹⁸ und R¹⁹ den obengenannten Umfang besitzen.

Besonders wichtig ist die Umsetzung von Verbindungen der Formel (VI), in der R¹⁴ für Wasserstoff steht, zum o-Nitrobenzaldehyd.

Als Reaktionsmedium wird ein aprotisches, polares Lösungsmittel oder Gemisch mehrerer von ihnen, eingesetzt. Solche aprotischen, polaren Lösungsmittel sind bevorzugt substituierte Säureamide, Ketone, Sulfoxide und Nitrile. Beispiele hierfür sind Dimethylformamid (DMF), Dimethylacetamid, Diethylformamid, Diethylacetamid, Hexamethylphosphorsäure-triamid, Aceton, Methyl-ethyl-keton, Methyl-tert.-butyl-keton, Dimethylsulfoxid, Acetonitril, Propionitril, N-Methyl-pyrrolidon (NMP), N-Methyl-caprolactam (NMC), N,H-Dimethyl-imidazolidinon (DMI). In besonders bevorzugter Weise werden die substituierten Säureamide, unter ihnen bevorzugt das DMF, eingesetzt. Das aprotische, polare Lösungsmittel kann durch ein inertes Lösungsmittel teilweise ersetzt werden. Solche inerten Lösungsmittel sind beispielsweise Benzol, Toluol, Xylol, Petrolether, Nitrotoluole, Dimethoxyethan, Methyl-tert.-butylether.

Der Ersatz kann in einer Menge von 0-50 Gew.-%, bezogen auf die gesamte Lösungsmittelmenge, vorgenommen werden.

In einer bevorzugten Variante des erfindungsgemäßen Verfahrens wird das substituierte β-Amino-styrol in Form des Rohprodukts aus der vorangegangenen Aminomethylenierung des zugrundeliegenden substituierten Toluols, wie es oben formelmäßig dargestellt wurde, eingesetzt. Wie aus dieser formelmäßigen Darstellung erkennbar ist, werden hierbei als Spaltprodukte substituierte Formamide vom Typ des DMF gebildet, die in dieser Form als erfindungsgemäß einzusetzendes aprotisches, polares Lösungsmittel benutzt werden.

Aus dem Reaktionsgemisch der vorangegangenen Aminomethylenierung werden im allgemeinen die Spaltprodukte primäres Alkanol und sekundäres Amin abdestilliert, jedoch ist das erfindungsgemäße Verfahren nicht so sensibel gegen geringe Mengen an Alkoholen und Aminen, daß eine solche destillative Entfernung unter hohem Aufwand vervollständigt werden müßte.

Das erfindungsgemäße Verfahren wird in Gegenwart einer Kupferverbindung, in bevorzugter Weise in Gegenwart eines wasserfreien Kupfersalzes, besonders bevorzugt in Gegenwart eines Cu(I)-Salzes, ganz besonders bevorzugt in Gegenwart eines Cu(I)-Halogenids in einer Menge von 1-200 Mol-%, bevorzugt 5-40 Mol-%, besonders bevorzugt 10-20 Mol-%, bezogen auf das substituierte β-Amino-styrol, durchgeführt. Die Kupferverbindung setzt sich zu Beginn der Reaktion wahrscheinlich mit Sauerstoff und möglicherweise dem Lösungsmittel zu einem Kupferkomplex um, dessen Natur nicht genau bekannt ist.

Das erfindungsgemäße Verfahren wird bei einer Temperatur von 0-120°C, bevorzugt 20-100°C, besonders bevorzugt 30-80°C durchgeführt. Als Sauerstoff kann reiner Sauerstoff, mit Sauerstoff angereicherte Luft oder die atmosphärische Luft selbst eingesetzt werden. Zur Minimierung der Abgase wird bevorzugt reiner Sauerstoff oder mit Sauerstoff angereicherte Luft eingesetzt.

Für den Fall, daß das erfindungsgemäße Verfahren mit der voranzustellenden Aminomethylenierung des zugrundeliegenden Toluols verbunden werden soll, wird beispielsweise so verfahren, daß man das zugrundeliegende Toluol mit elektronenanziehenden Substituenten in mindestens einer der ortho- und para-Stellungen, beispielsweise o-Nitrotoluol, mit einem ortho-Amid im 2-4-fachen molaren Überschuß in einem der genannten aprotischen, polaren Lösungsmittel oder einem inerten Lösungsmittel umsetzt. Ein solches ortho-Amid kann beispielsweise ein Aminalester, wie der tert.-Butyl- oder der Ethylaminalester, oder ein Säureamidacetal, beispielsweise DMF-Acetal, sein. Bei Einsatz der zuletztgenannten Säureamidacetale kann durch den Zusatz eines sekundären Amins, beispielsweise durch den Zusatz des oben formelmäßig dargestellten Pyrrolidins die Reaktionszeit verkürzt werden. Der entstandene Alkohol und das gegebenenfalls entstandene Amin werden weitgehend abdestilliert. Ausgehend vom zugrundeliegenden substituierten Toluol können hierbei Ausbeuten von über 95 %. erhalten werden.

Für die Durchführung des erfindungsgemäßen Verfahrens wird ein isoliertes substituiertes β-Amino-styrol oder das soeben beschriebene Rohprodukt aus der vorangehenden Aminomethylenierung eingesetzt. Hierzu können alle Reaktionspartner (Lösungsmittel, Cu-Verbindung und substituiertes β-Amino-styrol) vorgelegt werden oder es werden nur das Lösungsmittel und die Cu-Verbindung vorgelegt und das β-Amino-styrol in Lösung zugetropft. Das Reaktionsgemisch wird mit Sauerstoff oder einem sauerstoffhaltigen Gas durch eine Fritte oder eine andere Verteilungseinrichtung beschickt, wobei man die Temperatur im obengenannten Bereich hält.

Beide genannten Verfahrensvarianten (das substituierte β-Amino-styrol wird entweder vollständig vorgelegt oder im Verlaufe der Reaktion zudosiert) können auch unter Druck von beispielsweise 1,01-50 bar durchgeführt werden, wobei in der zweiten Variante das substituierte β-Amino-styrol zugepumpt wird.

Die Menge des aprotischen, polaren Lösungsmittels bzw. des oben beschriebenen Lösungsmittelgemisches mit einem inerten Lösungsmittel wird so bemessen, daß während der Umsetzung 100-10 000 ml, bevorzugt 100-1000 ml, besonders bevorzugt 10-500 ml Lösungsmittel pro 10 g des substituierten β-Amino-styrols vorliegen. Für den Fall, daß man in der beschriebenen zweiten Reaktionsvariante das substituierte β-Amino-styrol zudosiert, beispielsweise im Maße seiner Umsetzung, kann man demzufolge mit weniger Lösungsmittel auskommen als in der ersten Variante mit dem vollständigen Einsatz des substituierten β-Amino-styrols zu Beginn der Reaktion. In einer besonderen Art der 2. Variante wird das Cu(I)-halogenid anfangs nur teilweise zugesetzt und der Rest der vorgesehenen Menge im Laufe der Reaktion zudosiert.

Am Ende der Umsetzung, das durch das Verschwinden der roten Farbe des substituierten β-Amino-styrols sichtbar ist, wird das Lösungsmittel(gemisch) beispielsweise im Vakuum abdestilliert und der Rückstand in 3-8 n wäßrige Salzsäure gegeben. Der Benzaldehyd (I) fällt als schwach gefärbter Feststoff aus und hat nach dem Trocknen im allgemeinen bereits eine Reinheit von mehr als 95 %. Im Filtrat können 1-5 % Benzaldehyd (I) enthalten sein, die mit einem geeigneten Lösungsmittel, wie Methylenchlorid, extrahiert werden können.

Die Ausbeute beträgt bis zu 90 %, bezogen auf das substituierte β-Amino-styrol und bis zu 88 %, bezogen auf das zugrundeliegende Toluol, beispielsweise 80-91 % o-Nitrobenzaldehyd, bezogen auf das substituierte β-Amino-2-nitro-styrol und 85-89 %, bezogen auf das zugrundeliegende o-Nitrotoluol. Es ist möglich, den Reaktionsansatz vor der Aufarbeitung beispielsweise mit Pentan zu verdünnen und so einen Teil der katalytisch wirkenden Kupfer-Verbindung auszufällen, der erneut verwendet werden kann.

Die beschriebene hohe Ausbeute bei gleichzeitig hoher Reinheit im erfindungsgemäßen Verfahren ist überraschend, da mit einer Weiteroxidation des gebildeten Benzaldehyds (I) zur zugehörigen Benzoesäure gerechnet werden konnte.

### Beispiele

### Beispiel 1

In einer Destillationsapparatur wurden 20,6 g o-Nitrotoluol und 24 g Ethylaminalester in 50 ml DMF vorgelegt und 6 h auf 120°C zur Bildung des β-Amino-2-nitro-styrols erhitzt. Anschließend entfernte man das Ethanol durch kurzes Anlegen von Vakuum.

In einem zweiten Kolben wurden 2 g CuCl in 150 ml DMF vorgelegt und bei 65°C mit Sauerstoff begast. Nach 15 Min. tropfte man die Rohlösung des β-Amino-2-nitro-styrols innerhalb 3 h zu. 30 Min. nach Zugabeende konnte dünnschichtchromatographisch kein Edukt mehr nachgewiesen werden, und man engte am Rotationsverdampfer ein. Der Ansatz wurde anschließend in 100 ml 5 n HCl eingetragen, wobei o-Nitrobenzaldehyd als beigefarbener Stoff ausfiel. Aus dem Filtrat wurde durch Extrahieren mit Methylenchlorid weiteres Produkt isoliert.

Insgesamt betrug die Ausbeute 86,6 %, ausgehend von o-Nitrotoluol. Der ausgefallene o-Nitrobenzaldehyd hatte nach dem Trocknen eine Reinheit von 96,2 %. Die extrahierte Ware wurde über das Bisulfitaddukt gereinigt.

### Beispiel 2

Entsprechend Beispiel 1 wurde bei der Aminomethylenierung 0,15 Mol Pyrrolidin zugesetzt. Man erhielt ein Gemisch aus 86,7 % β-Pyrrolidino- und 13,3 % β-Dimethyl amino-2-nitrostyrol in DMF, das analog Beispiel 1 oxidiert wurde.

Die Ausbeute betrug 85,1 %, ausgehend von o-Nitro-toluol.

### Beispiel 3

Die Rohlösung von β-Dimethylamino-2-nitrostyrol, hergestellt analog Beispiel 1, wurde mit 250 ml DMF verdünnt und mit 3 g CuCl versetzt. Man begaste bei 50°C 45 Min. mit Sauerstoff und entfernte anschließend das Lösungsmittel. Nach der üblichen Aufarbeitung konnten 88,8 % o-Nitrobenzaldehyd, basierend auf dem eingesetzten o-Nitro-toluol, isoliert werden. Der Anteil an Nebenkomponenten im ausgefallenen Produkt betrug nur 0,8 %.

### Beispiel 4

50 mmol β-Morpholino-2-nitrostyrol und 1 g CuCl wurden in 120 ml DMSO vorgelegt und bei 60°C 90 min mit Sauerstoff begast. Nach Einengen und üblicher Aufarbeitung wurden 0,41 Mol o-Nitrobenzaldehyd isoliert.

### Beispiel 5

20 g β-Dimethylamino-2-nitrostyrol, 1 g CuCl und 300 ml DMF wurden in einem 300 ml-Autoklaven mit Begasungsrührer vorgelegt. Man heizte auf 50°C, drückte 10 bar Luft auf und hielt anschließend mit Sauerstoff einen Druck von 20 bar aufrecht. Nach 2 h wurde der Versuch abgebrochen und nach der Aufarbeitung 87,2 % o-Nitrobenzaldehyd erhalten.

### Beispiel 6

Analog Beispiel 1 wurde bei der Aminomethylierung 0,1 Mol N,N'-Dimethyl-ethylendiamin zugesetzt. Das erhaltene Styrolgemisch wurde mit 250 ml DMF verdünnt und nach Zugabe von 4 g CuCl 90 min bei 75°C mit Sauerstoff begast. Nach gaschromatographischer Analyse mit internem Standard waren 66,3 % o-Nitrobenzaldehyd entstanden.

### Beispiel 7

2,6 g β-Benzylamino-2-nitrostyrol wurden in 20 ml DMF gelöst und nach Zugabe von 0,3 g CuBr bei 50°C mit Sauerstoff begast. Nach 2 h konnte dünnschichtchromatographisch festgestellt werden, daß das Edukt sich umgesetzt hatte und o-Nitrobenzaldehyd entstanden war.

### Beispiel 8

8,6 g β-Dimethylamino-2-cyanostyrol wurden in 100 ml DMF gelöst. Man fügte 0,25 g CuCl zu und begaste 4 h bei 35-40°C mit Sauerstoff. Nach der üblichen wäßrigen Aufarbeitung wurde o-Cyanobenzaldehyd als Produkt erhalten.

## Patentansprüche

1. Verfahren zur Herstellung eines Benzaldehyds, der in mindestens einer der ortho- und para-Stellungen einen elektronenanziehenden Substituenten trägt, der Formel 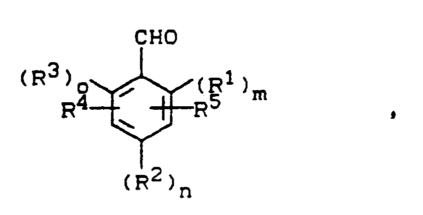 in der
R¹, R² und R³ unabhängig voneinander Nitro, Cyano, COOR⁶, SO₂-OR⁶ oder SO₂-R⁶ bedeuten,
R⁴ für Wasserstoff, geradkettiges oder verzweigtes C₁-C₈-Alkyl, geradkettiges oder verzweigtes C₁-C₈-Alkoxy, Phenyl, Benzyl, Cyclopentyl, Cyclohexyl, Fluor, Chlor, Brom oder -N(R⁶,R⁷) steht,
R⁵ Wasserstoff oder die Aldehydgruppe -CHO bedeutet,
wobei
R⁶ und R⁷ unabhängig voneinander geradkettiges oder verzweigtes C₁-C₄-Alkyl, Benzyl oder Phenyl bedeuten,
wobei weiterhin die genannten cyclischen Substituenten ihrerseits ein- oder zweimal durch Methyl, Ethyl, Methoxy, Ethoxy, Fluor, Chlor oder Brom substituiert sein können und
m, n und o unabhängig voneinander den Wert Null oder Eins annehmen können, die Summe m+n+o jedoch auf den Wert Eins oder Zwei beschränkt ist,
dadurch gekennzeichnet, daß man ein substituiertes β-Amino-styrol der Formel 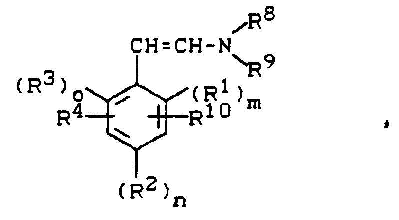 in der
R¹, R², R³, R⁴, m, n und o die genannte Bedeutung besitzen,
R⁸ und R⁹ unabhängig voneinander geradkettiges oder verzweigtes C₁-C₈-Alkyl, C₅-C₇-Cycloalkyl, geradkettiges oder verzweigtes C₂-C₈-Alkenyl, geradkettiges oder verzweigtes C₂-C₈-Alkoxyalkyl, geradkettiges oder verzweigtes C₃-C₈-Alkoxyalkenyl, C₆-C₁₂-Aryl, C₇-C₁₀-Aralkyl oder einen 5- bis 8-gliedrigen gesättigten oder ungesättigten heterocyclischen Ring, dessen Heteroatome ein oder zwei aus der Gruppe von N, O und S sind, bedeuten,
wobei weiterhin
R⁸ und R⁹ gemeinsam mit dem N-Atom, das sie substituieren, einen 5- bis 8-gliedrigen gesättigten oder ungesättigten, nichtaromatischen N-heterocyclischen Ring bilden können, der ein weiteres Heteroatom aus der Gruppe N, O oder S enthalten kann, und
R⁸ zusätzlich Wasserstoff bedeuten kann,
R⁹ zusätzlich die Gruppe 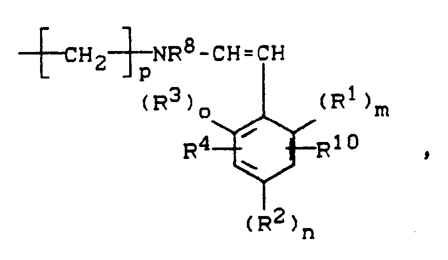
in der p den Wert 2, 3 oder 4 annimmt, bedeuten kann, und
R¹⁰ Wasserstoff oder die Gruppe 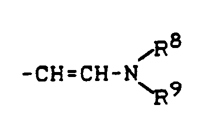
bedeutet, oder ein Gemisch mehrerer solcher β-Amino-styrole, die sich durch verschiedene Bedeutung von R⁸ und/oder R⁹ unterscheiden,
in der Lösung eines aprotischen, polaren Lösungsmittels bei 0-120°C, bevorzugt bei 20-100°C, besonders bevorzugt bei 30-80°C mit Sauerstoff in Gegenwart einer Cu-Verbindung umsetzt.

2. Verfahren nach Anspruch 1, dadurch gekennzeichnet, daß man ein substituiertes β-Amino-styrol der Formel 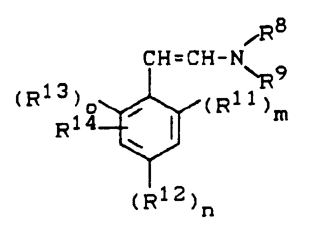 umsetzt, in der
R⁸, R⁹, m, n und o die in Anspruch 1 genannte Bedeutung haben,
R¹¹, R¹² und R¹³ unabhängig voneinander für Nitro oder Cyano stehen und
R¹⁴ Wasserstoff, geradkettiges oder verzweigtes C₁-C₄-Alkoxy, Fluor, Chlor oder Brom bedeutet.

3. Verfahren nach Anspruch 2, dadurch gekennzeichnet, daß man ein substituiertes β-Amino-styrol der Formel 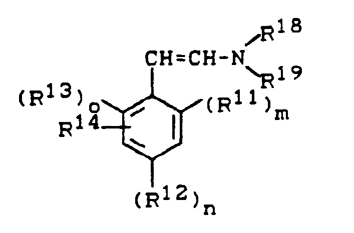 umsetzt, in der
R¹¹, R¹², R¹³, R¹⁴, m, n und o die in Anspruch 2 genannte Bedeutung besitzen und
R¹⁸ und R¹⁹ unabhängig voneinander geradkettiges oder verzweigtes C₁-C₈-Alkyl, Phenyl, Benzyl, Cyclohexyl oder einen 5-bis 6-gliedrigen gesättigten heterocyclischen Ring, der als Heteroatom ein N oder O enthält, bedeuten,
wobei weiterhin
R¹⁸ und R¹⁹ gemeinsam mit dem N-Atom, das sie substituieren, einen 5- bis 6-gliedrigen N-heterocyclischen Ring bilden können, der ein weiteres Heteroatom aus der Gruppe N und O enthalten kann.

4. Verfahren nach Anspruch 3, dadurch gekennzeichnet, daß man ein substituiertes β-Amino-styrol der Formel 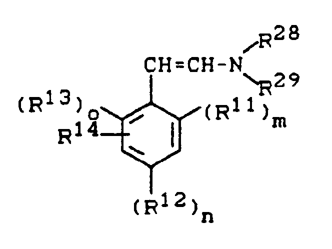 umsetzt, in der
R¹¹, R¹², R¹³, R¹⁴, m, n und o die in Anspruch 3 genannte Bedeutung besitzen und
R²⁸ und R²⁹ unabhängig voneinander geradkettiges oder verzweigtes C₁-C₄-Alkyl, Benzyl oder Cyclohexyl bedeuten,
wobei weiterhin
R²⁸ und R²⁹ gemeinsam mit den N-Atom das sie substituieren, den Pyrrolidinring, den Oxazolidinring, den Piperidinring, den Piperazinring oder den Morpholinring bilden können.

5. Verfahren nach Anspruch 3, dadurch gekennzeichnet, daß man ein substituiertes β-Amino-2-nitro-styrol der Formel 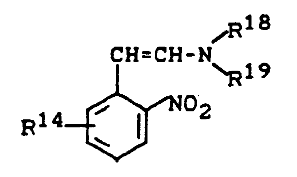 umsetzt, in der
R¹⁴, R¹⁸ und R¹⁹ den in Anspruch 3 genannten Umfang besitzen.

6. Verfahren nach Anspruch 1, dadurch gekennzeichnet, daß man das substituierte β-Amino-styrol in Form des Rohprodukts aus der Aminomethylenierung des zugrundeliegenden substituierten Toluols einsetzt.

7. Verfahren nach Anspruch 1, dadurch gekennzeichnet, daß als aprotisches, polares Lösungsmittel ein substituiertes Säureamid, ein Keton oder ein Sulfoxid, bevorzugt ein substituiertes Säureamid, besonders bevorzugt Dimethylformamid eingesetzt wird, das zu 5-50 Gew.-%, bezogen auf die gesamte Lösungsmittelmenge, durch ein inertes Lösungsmittel ersetzt sein kann.

8. Verfahren nach Anspruch 7, dadurch gekennzeichnet, daß als aprotisches, polares Lösungsmittel das aus der vorangegangenen Aminomethylenierung in Form des dabei erhaltenen rohen Reaktionsgemisches eingesetzt wird.

9. Verfahren nach Anspruch 1, dadurch gekennzeichnet, daß als Cu-Verbindung ein wasserfreies Kupfersalz, bevorzugt ein Cu(I)-Salz, besonders bevorzugt ein Cu(I)-Halogenid, eingesetzt wird.

10. Verfahren nach Anspruch 1, dadurch gekennzeichnet, daß die Cu-Verbindung in einer Menge von 1-200 Mol-%, bevorzugt 5-40 Mol-%, besonders bevorzugt 10-20 Mol-%, bezogen auf das substituierte β-Amino-styrol, eingesetzt wird.

## Claims

1. Process for preparing a benzaldehyde carrying an electron-withdrawing substituent in at least one of the ortho and para positions and having the formula 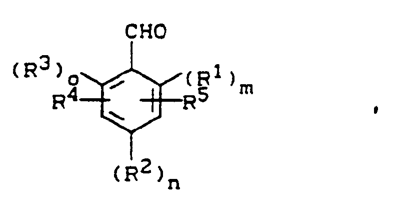 where
R¹, R² and R³, independently of one another, denote nitro, cyano, COOR⁶, SO₂-OR⁶ or SO₂-R⁶,
R⁴ represents hydrogen, straight-chain or branched C₁-C₈-alkyl, straight-chain or branched C₁-C₈-alkoxy, phenyl, benzyl, cyclopentyl, cyclohexyl, fluorine, chlorine, bromine or -N(R⁶, R⁷),
R⁵ denotes hydrogen or an aldehyde group -CHO,
R⁶ and R⁷, independently of one another, denote straight-chain or branched C₁-C₄-alkyl, benzyl or phenyl, the cyclic substituents mentioned may in turn be mono- or disubstituted by methyl, ethyl, methoxy, ethoxy, fluorine, chlorine or bromine, and
m, n and o, independently of one another, may be zero or one, the sum m+n+o being however restricted to the value one or two,
characterised in that a substituted β-amino-styrene of the formula 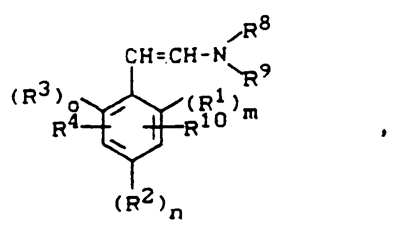 where
R¹, R², R³, R⁴, m, n and o have the meaning given,
R⁸ and R⁹, independently of one another, denote straight-chain or branched C₁-C₈-alkyl, C₅-C₇-cycloalkyl, straight-chain or branched C₂-C₈-alkenyl, straight-chain or branched C₂-C₈-alkoxyalkyl, straight-chain or branched C₃-C₈-alkoxyalkenyl, C₆-C₁₂-aryl, C₇-C₁₀-aralkyl or a 5- to 8-membered saturated or unsaturated heterocyclic ring whose heteroatoms are one or two heteroatoms from the group consisting of N, O and S,
or else
R⁸ and R⁹ together with the N atom which they substitute may be a 5- to 8-membered saturated or unsaturated, nonaromatic N-heterocyclic ring which may contain a further heteroatom from the group consisting of N, O and S,
R⁸ may be hydrogen,
R⁹ may be the group 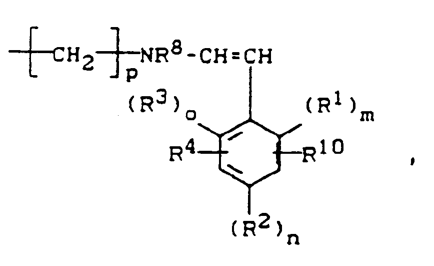
in which p is 2, 3 or 4, and
R¹⁰ is hydrogen or the group 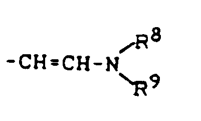
or a mixture of a plurality of such β-amino-styrenes which differ from each other in that the meaning of R⁸ and/or R⁹ is different,
is reacted in the solution of an aprotic, polar solvent at 0-120°C, preferably at 20-100°C, particularly preferably at 30-80°C, with oxygen in the presence of a Cu compound.

2. Process according to Claim 1, characterised in that the substituted β-amino-styrene used has the formula 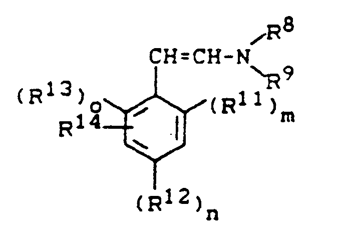 where
R⁸, R⁹, m, n and o have the meaning given in Claim 1,
R¹¹, R¹² and R¹³, independently of one another, represent nitro or cyano, and
R¹⁴ denotes hydrogen, straight-chain or branched C₁-C₄-alkoxy, fluorine, chlorine or bromine.

3. Process according to Claim 2, characterised in that the substituted β-amino-styrene used has the formula 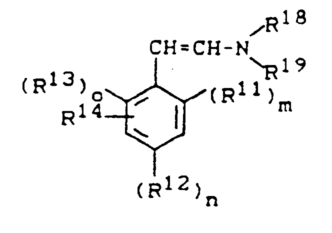 where
R¹¹, R¹², R¹³, R¹⁴, m, n and o have the meaning given in Claim 2 and
R¹⁸ and R¹⁹, independently of one another, denote straight-chain or branched C₁-C₈-alkyl, phenyl, benzyl, cyclohexyl or a 5- to 6-membered saturated heterocyclic ring which contains an N or O as the heteroatom,
or else
R¹⁸ and R¹⁹ together with the N atom which they substitute may be a 5- to 6-membered N-heterocyclic ring which may contain a further heteroatom from the group consisting of N and O.

4. Process according to Claim 3, characterised in that the substituted β-amino-styrene used has the formula 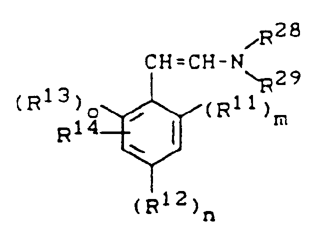 where
R¹¹, R¹², R¹³, R¹⁴, m, n and o have the meaning given in Claim 3 and
R²⁸ and R²⁹, independently of one another, denote straight-chain or branched C₁-C₄-alkyl, benzyl or cyclohexyl,
or else
R²⁸ and R²⁹ together with the N atom which they substitute may be a pyrrolidine ring, an oxazolidine ring, a piperidine ring, a piperazine ring or a morpholine ring.

5. Process according to Claim 3, characterised in that the substituted β-amino-2-nitrostyrene used has the formula 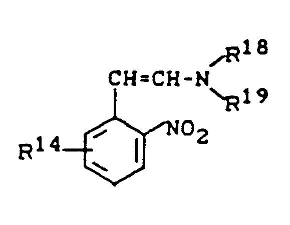 where
R¹⁴, R¹⁸ and R¹⁹ have the meanings mentioned in Claim 3.

6. Process according to Claim 1, characterised in that the substituted β-amino-styrene is used in the form of the crude product obtained from aminomethyleneation of the substituted toluene on which it is based.

7. Process according to Claim 1, characterised in that the aprotic, polar solvent used is a substituted amide, a ketone or a sulphoxide, preferably a substituted amide, particularly preferably dimethylformamide, 5-50% by weight of which, relative to the entire amount of solvent, may be replaced by an inert solvent.

8. Process according to Claim 7, characterised in that the aprotic, polar solvent used is the one obtained from the preceding aminomethyleneation in the form of the as-obtained crude reaction mixture.

9. Process according to Claim 1, characterised in that the Cu compound used is an anhydrous copper salt, preferably a Cu(I) salt, particularly preferably a Cu(I) halide.

10. Process according to Claim 1, characterised in that the Cu compound is used in an amount of 1-200 mol%, preferably 5-40 mol%, particularly preferably 10-20 mol%, relative to the substituted β-amino-styrene.

## Revendications

1. Procédé pour la préparation d'un benzaldéhyde qui porte, dans au moins une des positions ortho et para, un substituant attirant les électrons, répondant à la formule 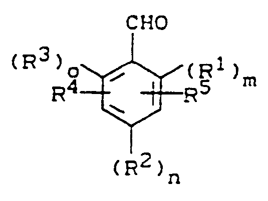 dans laquelle
R¹, R² et R³, indépendamment l'un de l'autre, représentent un groupe nitro, un groupe cyano, COOR⁶, SO₂-OR⁶ ou SO₂-R⁶,
R⁴ représente un atome d'hydrogène, un groupe alkyle en C₁-C₈ à chaîne droite ou ramifiée, un groupe alcoxy en C₁-C₈ à chaîne droite ou ramifiée, un groupe phényle, un groupe benzyle, un groupe cyclopentyle, un groupe cyclohexyle, un atome de fluor, un atome de chlore, un atome de brome ou -N(R⁶,R⁷),
R⁵ représente un atome d'hydrogène ou le groupe aldéhyde -CHO,
où
R⁶ et R⁷, indépendamment l'un de l'autre, représentent un groupe alkyle en C₁-C₄ à chaîne droite ou ramifiée, un groupe benzyle ou un groupe phényle,
où, en outre, les substituants cycliques mentionnés peuvent être, à leur tour, substitués une ou deux fois par un groupe méthyle, un groupe éthyle, un groupe méthoxy, un groupe éthoxy, un atome de fluor, un atome de chlore ou un atome de brome, et
m, n et o, indépendamment l'un de l'autre, peuvent prendre la valeur zéro ou un, la somme m+n+o étant toutefois limitée à la valeur un ou deux,
caractérisé en ce qu'on fait réagir un β-aminostyrène substitué de formule 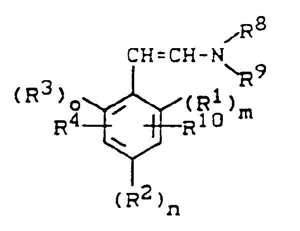 dans laquelle
R¹, R², R³, R⁴, m, n et o ont la signification mentionnée,
R⁸ et R⁹, indépendamment l'un de l'autre, représentent un groupe alkyle en C₁-C₈ à chaîne droite ou ramifiée, un groupe cycloalkyle en C₅-C₇, un groupe alcényle en C₂-C₈ à chaîne droite ou ramifiée, un groupe alcoxyalkyle en C₂-C₈ à chaîne droite ou ramifiée, un groupe alcoxyalcényle en C₃-C₈ à chaîne droite ou ramifiée, un groupe aryle en C₆-C₁₂, un groupe aralkyle en C₇-C₁₀ ou encore un noyau hétérocyclique penta- à octogonal saturé ou insaturé, dont les hétéroatomes représentent un ou deux éléments du groupe comprenant N, O et S,
où, en outre,
R⁸ et R⁹ peuvent former, conjointement avec l'atome de N qu'ils substituent, un noyau N-hétérocyclique non aromatique penta- à octogonal saturé ou insaturé, qui peut contenir un hétéroatome supplémentaire choisi parmi le groupe comprenant N, O ou S, et
R⁸ peut représenter, en outre, un atome d'hydrogène
R⁹ peut représenter, en outre, le groupe 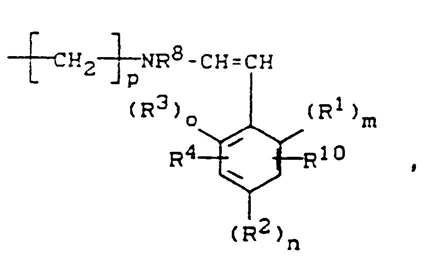
dans lequel p prend la valeur 2 , 3 ou 4, et
R¹⁰ représente un atome d'hydrogène ou le groupe 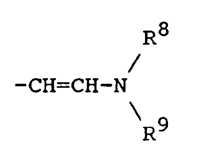
ou encore un mélange de plusieurs β-aminostyrènes de ce type qui se distinguent par des significations différentes de R⁸ et/ou de R⁹,
dans la solution d'un solvant polaire aprotique à 0-120°C, de préférence à 20-100°C, de manière particulièrement préférée à 30-80°C, avec de l'oxygène en présence d'un composé de Cu.

2. Procédé selon la revendication 1, caractérisé en ce qu'on fait réagir un β-aminostyrène substitué répondant à la formule 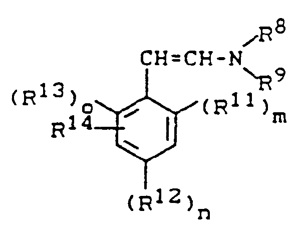 dans laquelle
R⁸, R⁹, m, n et o ont la signification indiquée dans la revendication 1,
R¹¹, R¹² et R¹³, indépendamment l'un de l'autre, représentent un groupe nitro ou un groupe cyano, et
R¹⁴ représente un atome d'hydrogène, un groupe alcoxy en C₁-C₄ à chaîne droite ou ramifiée, un atome de fluor, un atome de chlore ou un atome de brome.

3. Procédé selon la revendication 2, caractérisé en ce qu'on fait réagir un β-aminostyrène substitué de formule 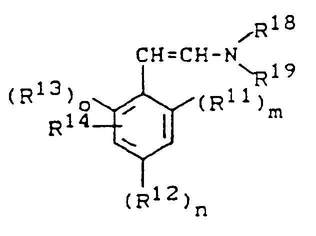 dans laquelle
R¹¹, R¹², R¹³, R¹⁴, m, n et o ont la signification mentionnée dans la revendication 2, et
R¹⁸ et R¹⁹, indépendamment l'un de l'autre, représentent un groupe alkyle en C₁-C₈ à chaîne droite ou ramifiée, un groupe phényle, un groupe benzyle, un groupe cyclohexyle ou encore un noyau hétérocyclique penta- à hexagonal saturé qui contient, à titre d'hétéroatome, un atome de N ou de O,
où, en outre
R¹⁸ et R¹⁹ peuvent former, conjointement avec l'atome de N qu'ils substituent, un noyau N-hétérocyclique penta- à hexagonal, qui peut contenir un hétéroatome supplémentaire choisi parmi le groupe comprenant N et O.

4. Procédé selon la revendication 3, caractérisé en ce qu'on fait réagir un β-aminostyrène substitué de formule 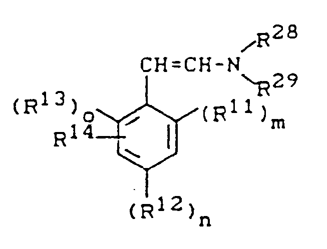 dans laquelle
R¹¹, R¹², R¹³, R¹⁴, m, n et o ont la signification mentionnée à la revendication 3, et
R²⁸ et R²⁹, indépendamment l'un de l'autre, représentent un groupe alkyle en C₁-C₄ à chaîne droite ou ramifiée, un groupe benzyle ou un groupe cyclohexyle,
où, en outre,
R²⁸ et R²⁹ peuvent former, conjointement avec l'atome de N qu'ils substituent, le noyau pyrrolidine, le noyau oxazolidine, le noyau pipéridine, le noyau pipérazine ou le noyau morpholine.

5. Procédé selon la revendication 3, caractérisé en ce qu'on fait réagir un β-amino-2-nitrostyrène substitué répondant à la formule 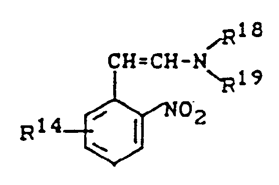 dans laquelle
R¹⁴, R¹⁸ et R¹⁹ ont la portée mentionnée à la revendication 3.

6. Procédé selon la revendication 1, caractérisé en ce qu'on met en oeuvre le β-aminostyrène substitué sous forme du produit brut provenant de l'aminométhylénation du toluène substitué de départ.

7. Procédé selon la revendication 1, caractérisé en ce qu'on met en oeuvre, à titre de solvant polaire aprotique, un amide d'acide substitué, une cétone ou un sulfoxyde, de préférence un amide d'acide substitué, de manière particulièrement préférée le diméthylformamide qui peut être remplacé à concurrence de 5-50% en poids, rapporté à la quantité totale de solvant, par un solvant inerte.

8. Procédé selon la revendication 7, caractérisé en ce qu'on met en oeuvre, à titre de solvant polaire aprotique, celui provenant de l'aminométhylénation qui a précédé, sous forme du mélange réactionnel brut obtenu en l'occurrence.

9. Procédé selon la revendication 1, caractérisé en ce qu'on met en oeuvre, à titre de composé de Cu, un sel de cuivre anhydre, de préférence un sel de Cu(I), de manière particulièrement préférée un halogénure de Cu(I).

10. Procédé selon la revendication 1, caractérisé en ce qu'on met en oeuvre le composé de Cu en une quantité de 1-200 moles %, de préférence de 5-40 moles %, de manière particulièrement préférée de 10-20 moles %, rapportée au β-aminostyrène substitué.
